# EUROPEAN PATENT APPLICATION

(11) **EP 4 574 184 A1**
(43) Date of publication of application: **25.06.2025**
(21) Application number: 24220197.8
(22) Date of filing: 16.12.2024
(51) Int. Cl.: A61M 1/02, A61M 1/36

(54) **LEUKOREDUCTION AND CONTINUOUS-FLOW CENTRIFUGATION OF WHOLE BLOOD**

(30) Priority: 18.12.2023 US 202363611312 P
(71) Applicant: Fenwal, Inc., Lake Zurich, IL 60047 (US)
(72) Inventor: KUSTERS, Benjamin E., Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

A blood processing device includes a pump system, a valve system, a continuous-flow centrifuge, and a controller programmed to control the operation of the pump system, the valve system, and the centrifuge to execute a blood separation procedure. The blood separation procedure executed by the controller includes pumping whole blood through a leukoreduction filter and into the centrifuge, separating the blood in the centrifuge into red blood cells and plasma, with an interface between the red blood cells and plasma located at an interface position within the centrifuge, and pumping at least a portion of the red blood cells and at least a portion of the plasma out of the centrifuge.

## Description

### Background

### Field of the Disclosure

The present disclosure relates to separation of whole blood. More particularly, the present disclosure relates to systems and procedures in which whole blood is conveyed through a leukoreduction filter and then separated using a continuous-flow centrifuge.

### Description of Related Art

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

According to one approach, whole blood may be separated into its constituents through centrifugation. This requires that the whole blood be passed through a centrifuge after it is withdrawn from, and before it is returned to, the blood source. To reduce contamination and possible infection (if the blood source is a human donor or patient), the blood is preferably processed within a sealed, sterile fluid flow circuit during the centrifugation process. The operator installs a fresh, sterile disposable flow circuit in the centrifuge before processing and removes and discards it afterwards. Typical disposable flow circuits are sealed and sterile, and include a separation chamber portion, which is mounted in cooperation on a durable, reusable assembly containing the hardware (centrifuge, drive system, pumps, valve actuators, programmable controller, and the like) that rotates the separation chamber and controls the flow through the fluid circuit. The separation chamber may be formed of a generally rigid material (e.g., molded plastic), in which case the chamber itself defines a flow path or channel in which blood is separated into two or more components, or a more flexible material (e.g., in the form of a belt or annulus), which relies upon the system hardware to support the chamber and define the shape of the chamber as blood flows through it.

With a disposable circuit loaded onto the centrifuge (or just prior to or during loading) the operator typically enters, for example, by means of a touch screen or other user interface system, a particular processing protocol to be executed by the system (e.g., a procedure wherein platelets are separated from whole blood and collected) and other parameters (e.g., the weight of the donor, the desired volume of separated blood component to be collected, etc.). When the system has been programmed, the operator phlebotomizes a donor and the system carries out the procedure, under the supervision of the operator.

The centrifuge rotates the separation chamber of the disposable flow circuit during processing, causing the heavier (greater specific gravity) components of the whole blood in the separation chamber, such as red blood cells, to move radially outwardly away from the center of rotation toward the outer or "high-G" wall of the separation chamber. The lighter (lower specific gravity) components, such as plasma, migrate toward the inner or "low-G" wall of the separation chamber. The boundary that forms between the heavier and lighter components in the separation chamber is commonly referred to as the interface. Various ones of these components can be selectively removed from the whole blood by providing appropriately located channeling structures and outlet ports in the flow circuit. For example, in one blood separation procedure, plasma is separated from cellular blood components and collected, with the cellular blood components and a replacement fluid being returned to the blood source. Alternatively, red blood cells may be harvested from the separation chamber and the rest of the blood constituents returned to the donor. Other processes are also possible including, without limitation, platelet collection, red blood cell exchanges, plasma exchanges, etc.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a blood processing device includes a pump system, a valve system, a continuous-flow centrifuge, and a controller programmed to control the operation of the pump system, the valve system, and the centrifuge to execute a blood separation procedure. The procedure executed by the controller includes pumping whole blood through a leukoreduction filter and into the centrifuge, separating the blood in the centrifuge into red blood cells and plasma (with an interface between the red blood cells and plasma located at an interface position within the centrifuge), and pumping at least a portion of the red blood cells and at least a portion of the plasma out of the centrifuge.

In another aspect, a blood separation method includes pumping whole blood through a leukoreduction filter and into a continuous-flow centrifuge. The blood in the centrifuge is separated into red blood cells and plasma, with an interface between the red blood cells and plasma located at an interface position within the centrifuge. At least a portion of the red blood cells and at least a portion of the plasma is pumped out of the centrifuge.

### Brief Description of the Drawings

Fig. 1 is a perspective view of an exemplary reusable hardware component of a blood processing system which is configured to receive a disposable fluid flow circuit;
Fig. 2 is a plan view of an exemplary disposable fluid flow circuit for use in combination with the durable hardware component of Fig. 1;
Fig. 3 is a schematic view of the fluid flow circuit of Fig. 2 mounted to the processing device of Fig. 1 to complete a blood processing system according to an aspect of the present disclosure;
Fig. 4 is a schematic view of the blood processing system of Fig. 3 executing a "blood prime" stage of an exemplary blood processing procedure;
Fig. 5 is a schematic view of the blood processing system of Fig. 3 executing an "establish separation" stage of an exemplary blood processing procedure;
Fig. 6 is a schematic view of the blood processing system of Fig. 3 executing a "collection" stage of an exemplary blood processing procedure, with whole blood being leukoreduced before being centrifugally separated;
Fig. 7 is a schematic view of the blood processing system of Fig. 3 executing a variation of the "collection" stage of Fig. 6, with whole blood bypassing a leukoreduction filter before being centrifugally separated;
Fig. 8 is a schematic view of the blood processing system of Fig. 3 executing an "air flush" stage of an exemplary blood processing procedure;
Fig. 9 is a schematic view of the blood processing system of Fig. 3 executing an "air evacuation" stage of an exemplary blood processing procedure; and
Fig. 10 is a schematic view of the blood processing system of Fig. 3 executing a "sealing" stage of an exemplary blood processing procedure.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

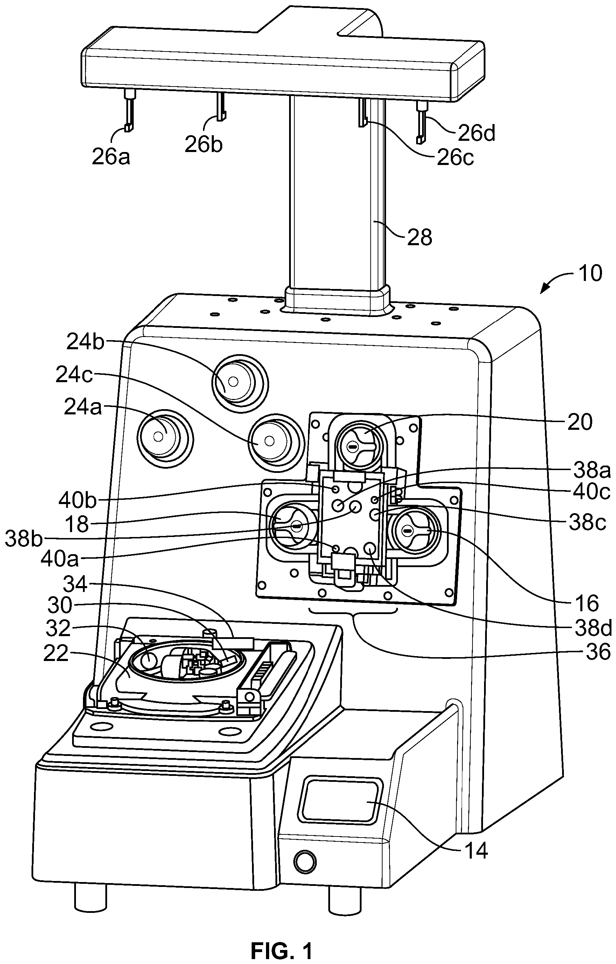

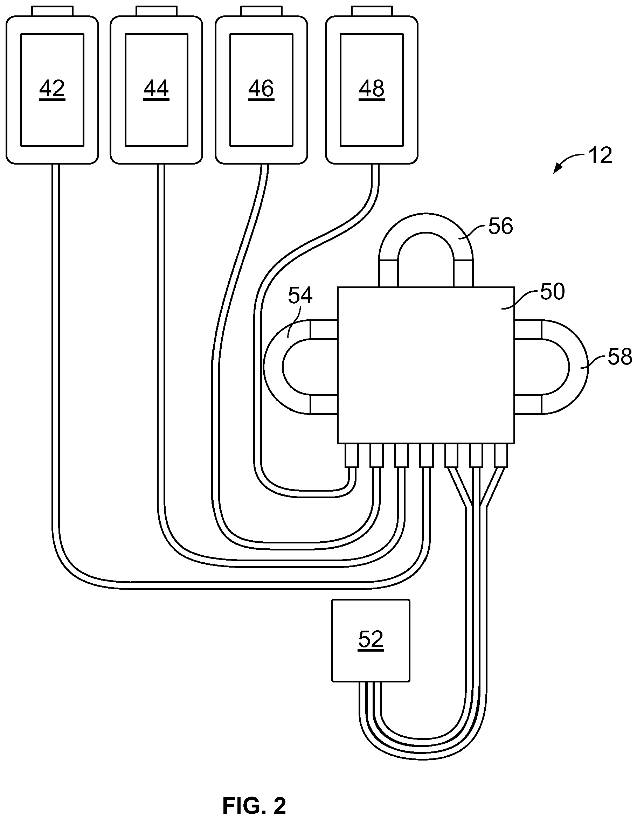

Fig. 1 depicts a reusable hardware component or processing device of a blood processing system, generally designated 10, while Fig. 2 depicts a disposable fluid flow circuit, generally designated 12, to be used in combination with the processing device 10 for processing blood. The processing device 10 and fluid flow circuit 12 are comparable to the ones shown and described in greater detail in PCT Patent Application Publication No. WO 2021/194824 A1 (which is hereby incorporated herein by reference), though it should be understood that a processing device and fluid flow circuit according to the present disclosure may be variously configured without departing from the scope of the present disclosure. The illustrated processing device 10 includes associated pumps, valves, sensors, displays, and other apparatus for configuring and controlling flow of fluid through the fluid flow circuit 12, described in greater detail below. The blood processing system may be directed by a controller integral with the processing device 10 that includes a programmable microprocessor to automatically control the operation of the pumps, valves, sensors, etc. The processing device 10 may also include wireless communication capabilities to enable the transfer of data from the processing device 10 to the quality management systems of the operator.

More specifically, the illustrated processing device 10 includes a user input and output touchscreen 14, a pump station including a first pump 16 (for pumping, e.g., whole blood), a second pump 18 (for pumping, e.g., plasma) and a third pump 20 (for pumping, e.g., additive solution), a centrifuge mounting station and drive unit 22 (which may be referred to herein as a "centrifuge"), and clamps 24a-c. The touchscreen 14 enables user interaction with the processing device 10, as well as the monitoring of procedure parameters, such as flow rates, container weights, pressures, etc. The pumps 16, 18, and 20 (collectively referred to herein as being part of a "pump system" of the processing device 10) are illustrated as peristaltic pumps capable of receiving tubing or conduits and moving fluid at various rates through the associated conduit dependent upon the procedure being performed. An exemplary centrifuge mounting station/drive unit is seen in U.S. Patent No. 8,075,468 (with reference to Figs. 26-28), which is hereby incorporated herein by reference. The clamps 24a-c (collectively referred to herein as being part of the "valve system" of the processing device 10) are capable of opening and closing fluid paths through the tubing or conduits and may incorporate RF sealers in order to complete a heat seal of the tubing or conduit placed in the clamp to seal the tubing or conduit leading to a product container upon completion of a procedure.

Sterile connection/docking devices may also be incorporated into one or more of the clamps 24a-c. The sterile connection devices may employ any of several different operating principles. For example, known sterile connection devices and systems include radiant energy systems that melt facing membranes of fluid flow conduits, as in U.S. Patent No. 4,157,723; heated wafer systems that employ wafers for cutting and heat bonding or splicing tubing segments together while the ends remain molten or semi-molten, such as in U.S. Patent Nos. 4,753,697; 5,158,630; and 5,156,701; and systems employing removable closure films or webs sealed to the ends of tubing segments as described, for example, in U.S. Patent No. 10,307,582. Alternatively, sterile connections may be formed by compressing or pinching a sealed tubing segment, heating and severing the sealed end, and joining the tubing to a similarly treated tubing segment as in, for example, U.S. Patent Nos. 10,040,247 and 9,440,396. All of the above-identified patents are incorporated by reference in their entirety. Sterile connection devices based on other operating principles may also be employed without departing from the scope of the present disclosure.

The processing device 10 also includes hangers 26a-d (which may each be associated with a weight scale) for suspending the various containers of the disposable fluid circuit 12. The hangers 26a-d are preferably mounted to a support 28, which is vertically translatable to improve the transportability of the processing device 10. An optical system comprising a laser 30 and a photodetector 32 is associated with the centrifuge 22 for determining and controlling the location of an interface between separated blood components within the centrifuge 22. An exemplary optical system is shown in U.S. Patent Application Publication No. 2019/0201916, which is hereby incorporated herein by reference. An optical sensor 34 is also provided to optically monitor one or more conduits leading into or out of the centrifuge 22.

The face of the processing device 10 includes a nesting module 36 for seating a flow control cassette 50 (Fig. 2) of the fluid flow circuit 12 (described in greater detail below). The cassette nesting module 36 is configured to receive various disposable cassette designs so that the system may be used to perform different types of procedures. Embedded within the illustrated cassette nesting module 36 are four valves 38a-d (collectively referred to herein as being part of the "valve system" of the processing device 10) for opening and closing fluid flow paths within the flow control cassette 50, and three pressure sensors 40a-c capable of measuring the pressure at various locations of the fluid flow circuit 12.

With reference to Fig. 2, the illustrated fluid flow circuit 12 includes a plurality of containers 42, 44, 46, and 48, with a flow control cassette 50 and a processing/separation chamber 52 that is configured to be received in the centrifuge 22, all of which are interconnected by conduits or tubing segments, so as to permit continuous flow centrifugation. The flow control cassette 50 routes the fluid flow through three tubing loops 54, 56, 58, with each loop being positioned to engage a particular one of the pumps 16, 18, 20. The conduits or tubing may extend through the cassette 50, or the cassette 50 may have pre-formed fluid flow paths that direct the fluid flow.

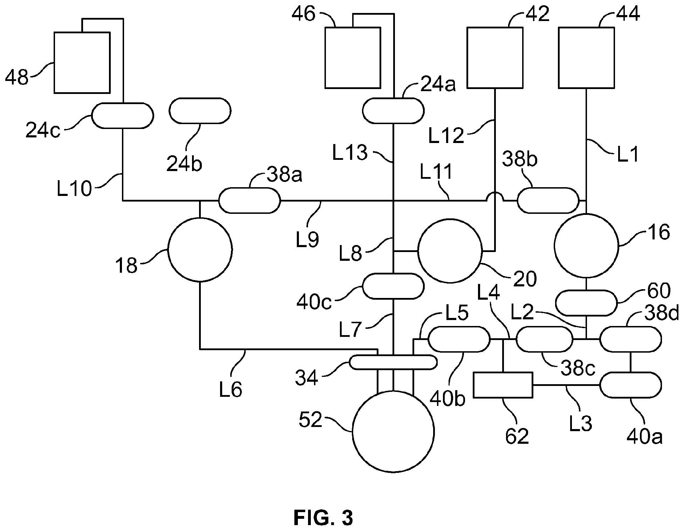

In the fluid flow circuit 12 shown in Fig. 2, container 42 may be prefilled with additive solution, container 44 may be filled with whole blood and connected to the fluid flow circuit 12 at the time of use, container 46 may be an empty container for the receipt of red blood cells separated from the whole blood, and container 48 may be an empty container for the receipt of plasma separated from the whole blood. While Fig. 2 shows a whole blood container 44 (configured as a blood pack unit, for example) as a blood source and the principles described herein may be most applicable when processing previously collected blood, it is within the scope of the present disclosure for the blood source to be a living donor. The fluid flow circuit 12 may include an air trap 60 (Fig. 3) and does include a leukoreduction filter 62 through which the whole blood is flowed prior to entering the processing chamber 52.

The processing chamber 52 may be pre-formed in a desired shape and configuration by injection molding from a rigid plastic material, as shown and described in U.S. Patent No. 6,849,039, which is hereby incorporated herein by reference. The specific geometry of the processing chamber 52 may vary depending on the elements to be separated, and the present disclosure is not limited to the use of any specific chamber design. For example, it is within the scope of the present disclosure for the processing chamber 52 to be configured formed of a generally flexible material, rather than a generally rigid material. When the processing chamber 52 is formed of a generally flexible material, it relies upon the centrifuge 22 to define a shape of the processing chamber 52. An exemplary processing chamber formed of a flexible material and an associated centrifuge are described in U.S. Patent No. 6,899,666, which is hereby incorporated herein by reference.

The controller of the processing device 10 may be pre-programmed to automatically operate the system to perform one or more standard blood processing procedures selected by an operator by input to the touchscreen 14, and configured to be further programmed by the operator to perform additional blood processing procedures. The controller may be pre-programmed to substantially automate a wide variety of procedures, including, but not limited to: red blood cell and plasma production from a single unit of whole blood, buffy coat pooling, buffy coat separation into a platelet product (as described in U.S. Patent Application Publication No. 2018/0078582, which is hereby incorporated herein by reference), glycerol addition to red blood cells, red blood cell washing, platelet washing, and cryoprecipitate pooling and separation.

The pre-programmed blood processing procedures operate the system at pre-set settings for flow rates and centrifugation forces, and the programmable controller may be further configured to receive input from the operator as to one or more of flow rates and centrifugation forces for the standard blood processing procedure to override the pre-programmed settings. In addition, the programmable controller may be configured to receive input from the operator through the touchscreen 14 for operating the system to perform a non-standard blood processing procedure.

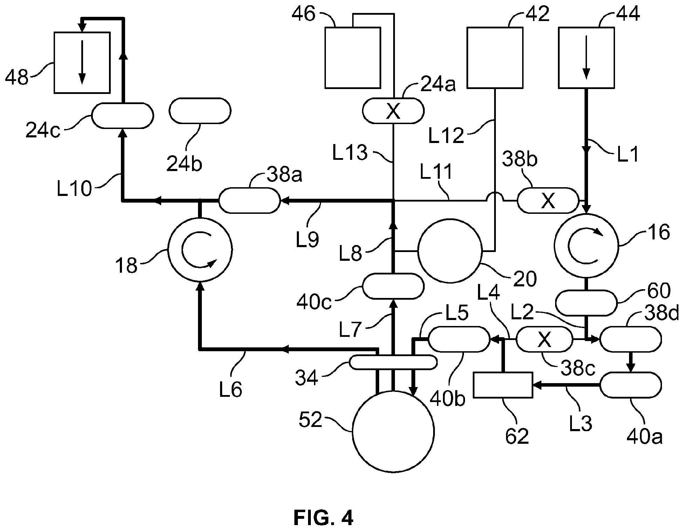

In an exemplary procedure, the processing device 10 and the fluid flow circuit 12 may be used in combination to process a unit of whole blood into a red blood cell product and a plasma product. Fig. 3 is a schematic illustration of the fluid flow circuit 12 mounted to the processing device 10, with selected components of the fluid flow circuit 12 and selected components of the processing device 10 being shown. Figs. 4-10 show different stages of an exemplary procedure. As shown in Figs. 4-10, one of the clamps 24b is not used in producing the red blood cell and plasma products (but would be used in other procedures), while the other illustrated components of the processing device 10 are employed.

In an initial stage, which is referred to herein as a "blood prime" stage and shown in Fig. 4, selected components of the fluid flow circuit 12 are primed using blood from a blood source. This is in contrast to typical apheresis devices, which employ a separately provided fluid (e.g., anticoagulant or saline) to prime a fluid flow circuit. The blood source is shown in Fig. 4 as the whole blood container 44, but may alternatively be a living donor. Thus, it should be understood that the term "whole blood" may refer to blood that either includes or omits an anticoagulant fluid. While Fig. 4 illustrates use of whole blood to prime selected components of the fluid flow circuit 12, it should be understood that other fluids (e.g., saline or some other non-biological fluid) may be used to prime components of the fluid flow circuit 12.

During the blood prime stage, whole blood is drawn into the fluid flow circuit 12 from the blood source (the whole blood container 44 in the embodiment of Fig. 4) via line L1 by operation of the first pump 16 (which may be referred to as the "whole blood pump"). Valves 38b and 38c are closed, which directs the blood into line L2, through air trap 60, and into line L3. The blood flows through open valve 38d, through pressure sensor 40a (which measures the pressure of the leukoreduction filter 62), through the leukoreduction filter 62, and into line L5. The blood in line L5 flows through the pressure sensor 40b (which measures the pressure of the processing chamber 52), through the optical sensor 34, and then into the processing chamber 52, which is positioned within the centrifuge 22 of the processing device 10. In one embodiment, valve 38c may be temporarily opened (either in combination with valve 38d remaining open or being temporarily closed) in order to direct some of the blood from line L2 into line L4, through open valve 38c, and into line L5, where it flows through pressure sensor 40b and through the optical sensor 34 before flowing into the processing chamber 52.

The centrifuge 22 may be stationary during the blood prime stage or may instead be controlled by the controller of the processing device 10 to spin at a low rotation rate (e.g., on the order of approximately 1,000-2,000 rpm). It may be advantageous for the centrifuge 22 to rotate during the blood prime stage in order to create enough g-force to ensure that the air in the processing chamber 52 (which includes air already present in the processing chamber 52, along with air moved into the processing chamber 52 from lines L1-L4 and/or the leukoreduction filter 62 by the flow of blood) is forced towards the low-G (radially inner) wall of the processing chamber 52. Higher centrifuge rotation rates, such as 4,500 rpm (which is required for steady state separation, as will be described) may be undesirable as air blocks (in which air gets stuck and cannot be forced out of the processing chamber 52, causing pressure to rise) are more likely at higher g-forces.

The blood entering the processing chamber 52 will move towards the high-G (radially outer) wall of the processing chamber 52, displacing air towards the low-G wall. A plasma outlet port of the processing chamber 52 is associated with the low-G wall of the processing chamber 52, such that most of the air will exit the processing chamber 52 via the plasma outlet port and associated line L6, although some air may also exit processing chamber 52 via a red blood cell outlet port associated with the high-G wall of the processing chamber 52.

Clamp 24a and valve 38b are closed, while the second pump 18 (which may be referred to as the "plasma pump) is active and the third pump 20 (which may be referred to as the "additive pump") is inactive. Such an arrangement will direct the air exiting the processing chamber 52 via the red blood cell outlet port through associated line L7 and pressure sensor 40b, into line L8 and then into line L9. Valve 38a is open, such that the air flowing through line L9 will meet up with the air flowing through line L6 (i.e., the air that exits the processing chamber 52 via the plasma outlet port). The combined air will flow through line L10 and open clamp 24c, into the plasma collection container 48. It should be understood that, in Figs. 4-10, arrows on the containers represent the direction of fluid flow between the container and the conduit connected to the container. For example, line L10 is shown as being connected to the top of the plasma collection container 48, such that a downward arrow (as in Fig. 4) represents downward fluid flow into the plasma collection container 48. In contrast, line L1 is shown as being connected to the bottom of the whole blood container 44, such that a downward arrow (as in Fig. 4) represents downward fluid flow out of the whole blood container 44.

The flow of air out of the processing chamber 52 via either outlet port is monitored by the optical sensor 34, which is capable of determining the optical density of the fluid flowing through the monitored lines and discerning between air and a non-air fluid in lines L6 and L7. When a non-air fluid is detected in both lines L6 and L7, the controller of the processing device 10 will end the blood prime stage and move on to the next stage of the procedure. The amount of blood drawn into the fluid flow circuit 12 from the blood source during the blood prime stage will vary depending on a number of factors (e.g., the amount of air in the fluid flow circuit 12), but may be on the order of approximately 50 to 100 mL. The blood prime stage may take on the order of one to two minutes.

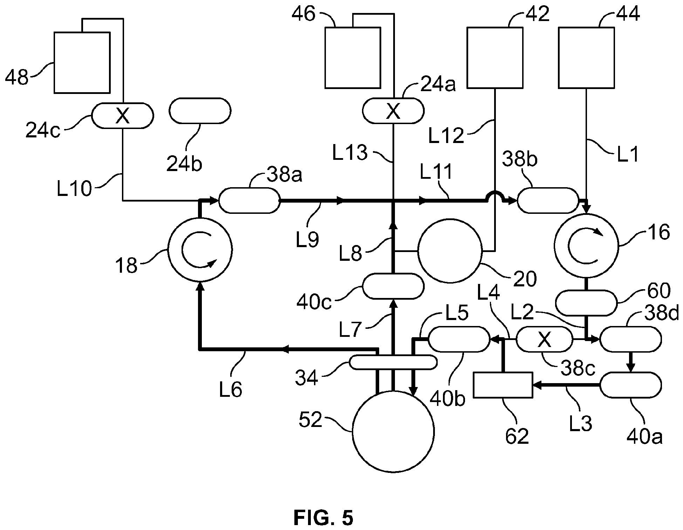

The next stage (shown in Fig. 5) is referred to herein as the "establish separation" stage. Once non-air fluid has been detected in lines L6 and L7, the rotational speed of the centrifuge 22 will be increased to a rate that is sufficient to separate blood into packed red blood cells and platelet-poor plasma (which may be in the range of approximately 4,500 to 5,500 rpm, for example). As for the whole blood pump 16, it continues to operate, but no additional blood is drawn into the fluid flow circuit 12 from the blood source during the establish separation stage.

As the blood source includes (in the case of a whole blood container) or provides (in the case of a living donor) only a single unit of whole blood (approximately 500 mL), the system must work with a finite fluid volume. To avoid product loss or quality issues, the plasma and red blood cells initially separated from the blood in the processing chamber 52 and removed from the processing chamber 52 are not directed to their respective collection containers, but are instead mixed together to form recombined whole blood and recirculated back into the processing chamber 52.

More particularly, during the establish separation stage, separated plasma will exit the processing chamber 52 via the plasma outlet port and associated line L6. Clamp 24c is closed during this stage, while valve 38a remains open, which directs the plasma from line L6 into line L9. Separated red blood cells exit the processing chamber 52 via the red blood cell outlet port and associated line L7. In the illustrated embodiment, there is no pump associated with line L7, such that the red blood cells exit the processing chamber 52 at a rate that is equal to the difference between the rate of the whole blood pump 16 and the rate of the plasma pump 18. In alternative embodiments, there may be a pump associated with the red blood cell outlet line instead of the plasma outlet line or a first pump associated with the plasma outlet line and a second pump associated with the red blood cell outlet line.

The additive pump 20 is inactive during this stage, thereby directing the red blood cells from line L7 into line L8. The plasma flowing through line L9 is mixed with the red blood cells flowing through line L8 at a junction of the two lines L8 and L9 to form recombined whole blood. Clamp 24a is closed, which directs the recombined whole blood into line L11 and through now-open valve 38b. The whole blood pump 16 draws the recombined whole blood into line L2 from line L11 (rather than drawing additional blood into the fluid flow circuit 12 from the blood source), with the recombined blood passing through air trap 60, open valve 38d, pressure sensor 40a, leukoreduction filter 62, pressure sensor 40b, and optical sensor 34 before flowing back into the processing chamber 52, where it is again separated into plasma and red blood cells.

As the blood circulating through the fluid flow circuit 12 during the establish separation stage passes through the leukoreduction filter 62, white blood cells will be continuously removed from the blood by the leukoreduction filter 62 throughout the establish separation stage. The pressure sensor 40a functions to monitor the pressure of the leukoreduction filter 62 to ensure that it does not become blocked with white blood cells. As a relatively small amount of blood is typically circulated through the fluid flow circuit 12 during the establish separation stage (and during the preceding blood prime stage), it is unlikely that the leukoreduction filter 62 will become blocked at this time.

The establish separation stage continues until steady state separation has been achieved, which may take on the order of approximately one to two minutes. As used herein, the phrase "steady state separation" refers to a state in which blood is separated into its constituents in the processing chamber 52, with the radial position of the interface between separated components within the processing chamber 52 being at least substantially maintained (rather than moving radially inwardly or outwardly). The position of the interface may be determined and controlled according to any suitable approach, including using an interface detector of the type described in U.S. Patent Application Publication No. 2019/0201916.

Preferably, steady state separation is achieved with the interface between separated components within the processing chamber 52 at a target location. The target location may correspond to the location of the interface at which separation efficiency is optimized, with the precise location varying depending on a number of factors, such as the hematocrit of the whole blood. Another factor is whether the leukoreduction filter 62 is configured to remove platelets along with removing white blood cells from the whole blood before it enters the processing chamber 52. When the leukoreduction filter 62 is configured to remove both platelets and white blood cells, the interface may be positioned relatively close to the low-G wall (compared to blood that has not been filtered before being centrifugally separated) because there will be no risk of platelets flowing out of the plasma outlet port with the platelet-poor plasma. It will, thus, be seen that the leukoreduction filter 62 (when configured to remove both platelets and white blood cells from whole blood entering the processing chamber 52) allows for more "aggressive" plasma selection and increased plasma product volumes (on account of the processing chamber 52 not being responsible for separating platelets from the plasma).

In the illustrated embodiment, the position of the interface within the processing chamber 52 may be adjusted by changing the flow rate of the plasma pump 18, with the flow rate being increased to draw more separated plasma out of the processing chamber 52 (which decreases the thickness of the plasma layer within the processing chamber 52) and move the interface toward the low-G wall or decreased to draw less plasma out of the processing chamber 52 (which increases the thickness of the plasma layer within the processing chamber 52) and move the interface toward the high-G wall. In an exemplary procedure, the controller of the processing device 10 will control the whole blood pump 16 to operate at a constant rate, with the plasma pump 18 initially operating at the same rate, which will quickly increase the thickness of the red blood cell layer within the processing chamber 52 and move the interface toward the low-G wall. The rate of the plasma pump 18 is gradually decreased as the thickness of the red blood cell layer increases and the location of the interface approaches the target location. As described above, the target location of the interface may depend upon the hematocrit of the whole blood, meaning that the rate of the plasma pump 18 (which controls the position of the interface) may also depend on the hematocrit of the whole blood. In one embodiment, this relationship may be expressed as follows: Theoretical plasma pump rate = whole blood pump rate - ((whole blood hematocrit * whole blood pump rate)/hematocrit of separated red blood cells)

The hematocrit of the whole blood may be measured before the procedure begins or by the optical sensor 34 during the procedure, while the hematocrit of the separated red blood cells may be determined during the procedure by the optical sensor 34 monitoring line L7. In practice, the plasma pump rate will typically not remain at the theoretical rate once steady state separation has been achieved, with the interface at the target location, but rather the plasma pump rate will instead tend to "flutter" around the theoretical rate.

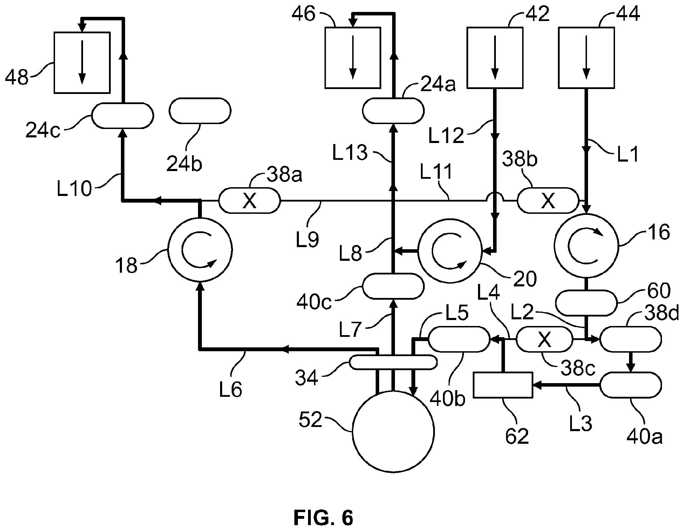

Regardless of the particular manner in which the controller of the processing device 10 executes the establish separation stage and arrives at steady state separation, once steady state separation has been established, the controller ends the establish separation stage and advances the procedure to a "collection" stage, which is illustrated in Fig. 6. At the beginning of the collection stage, the centrifuge 22, the whole blood pump 16, and the plasma pump 18 all continue operating at the same rates at which they were operating at the end of the establish separation stage. The valve system of the processing device 10, however, is adjusted to direct the separated plasma and red blood cells to their respective collection containers (rather than recombining them and recirculating them through the centrifuge 22), while causing additional blood to be drawn into the fluid flow circuit 12 from the blood source until a total of one unit of whole blood has been drawn into the fluid flow circuit 12.

More particularly, during the collection stage, valve 38b is closed, which causes the whole blood pump 16 to draw additional blood into line L1 from the blood source (which is the whole blood container 44 in the illustrated embodiment, but may be a living donor). The whole blood pump 16 draws the blood from the blood source into line L2 from line L1, with the blood passing through air trap 60, open valve 38d, pressure sensor 40a, leukoreduction filter 62, pressure sensor 40b, and optical sensor 34 before flowing into the processing chamber 52, where it is separated into plasma and red blood cells. As for the platelets and the white blood cells of the whole blood, they will be retained in the leukoreduction filter 62 without ever reaching the processing chamber 52 unless the leukoreduction filter 62 is configured as a platelet-sparing filter, in which case the white blood cells will be retained in the leukoreduction filter 62 while the majority of the platelets will remain in the processing chamber 52 during the collection stage.

The separated plasma exits the processing chamber 52 via the plasma outlet port and associated line L6. Valve 38a is closed, which directs the plasma from line L6 into line L10, through open clamp 24c, and into the plasma collection container 48.

As for the separated red blood cells, they exit the processing chamber 52 via the red blood cell outlet port and associated line L7. The additive pump 20 is operated by the controller to draw an additive solution (which is ADSOL^{®} in one exemplary embodiment, but may be some other red blood cell additive) from the additive solution container 42 via line L12. The red blood cells flowing through line L7 are mixed with the additive solution flowing through line L12 at a junction of the two lines L7 and L12 to form a mixture that continues flowing into and through line L8. Valves 38a and 38b are closed, while clamp 24a is open, which directs the mixture into line L13, through open clamp 24a, and into the red blood cell collection container 46.

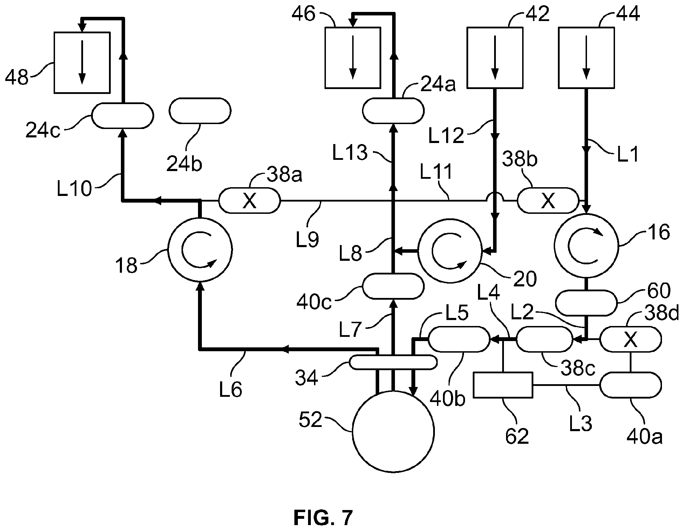

As described above, pressure sensor 40a monitors the leukoreduction filter 62 to determine whether it has become blocked with leukocytes from the whole blood flowing therethrough. If the controller of the processing device 10 determines that the leukoreduction filter 62 has become blocked during the default collection stage of Fig. 6 (based on elevated signals received by the controller from the pressure sensor 40a), the controller may implement an alternative version of the collection stage, which is illustrated in Fig. 7. In the configuration of Fig. 7 (in which the whole blood from the blood source is not leukoreduced before being separated in the centrifuge 22), valve 38d is closed, while valve 38c is open, which directs the whole blood from line L2 into line L4 and then into line L5, bypassing the leukoreduction filter 62. The unfiltered blood flows through pressure sensor 40b and through optical sensor 34 before entering the processing chamber 52, where it continues to be separated into red blood cells and plasma, which are subsequently directed to their respective collection containers 46 and 48 (as in the version of the collection stage shown in Fig. 6). The controller of the processing device 10 may alert an operator that the collected red blood cells have not been leukoreduced.

In one embodiment, the operational rate of one or more of the pumps may be different during the collection stages shown in Figs. 6 and 7. As explained above, when platelets have been removed from the whole blood by the leukoreduction filter 62 (as may be the case in the collection stage shown in Fig. 6), the position of the interface within the processing chamber 52 may be relatively close to the low-G wall. However, when the whole blood bypasses the leukoreduction filter 62 (as in the collection stage shown in Fig. 7), platelets will be introduced into the processing chamber 52. Accordingly, in order to prevent the platelets from exiting the processing chamber 52 with the separated plasma via the plasma outlet port, it may be advantageous to adjust the operation of one or more of the pumps during the collection stage shown in Fig. 7 so as to move the interface away from the low-G wall of the processing chamber 52. This may be implemented by reducing the operational rate of the plasma pump 18 and/or increasing the operational rate of the additive pump 20, for example.

Regardless of whether the whole blood is fully or only partially leukoreduced, the collection stage continues until one unit of whole blood has been drawn into the fluid flow circuit 12 from the blood source. In the case of a whole blood container 44 being used as a blood source (as in the illustrated embodiment) the collection stage will end when the whole blood container 44 (which is initially provided with one unit of whole blood) is empty, with different approaches possibly being employed to determine when the whole blood container 44 is empty. For example, in one embodiment, a pressure sensor (not illustrated) monitors the hydrostatic pressure of the whole blood container 44. An empty whole blood container 44 may be detected when the hydrostatic pressure measured by pressure sensor is at or below a threshold value. Alternatively (or additionally), the weight of the whole blood container 44 may be monitored by a weight scale, with an empty whole blood container 44 being detected when the weight is at or below a threshold value. In the case of a living donor (or in the event that the whole blood container 44 is provided with more than one unit of blood), the volumetric flow rate of the whole blood pump 16 may be used to determine when one unit of whole blood has been drawn into the fluid flow circuit 12.

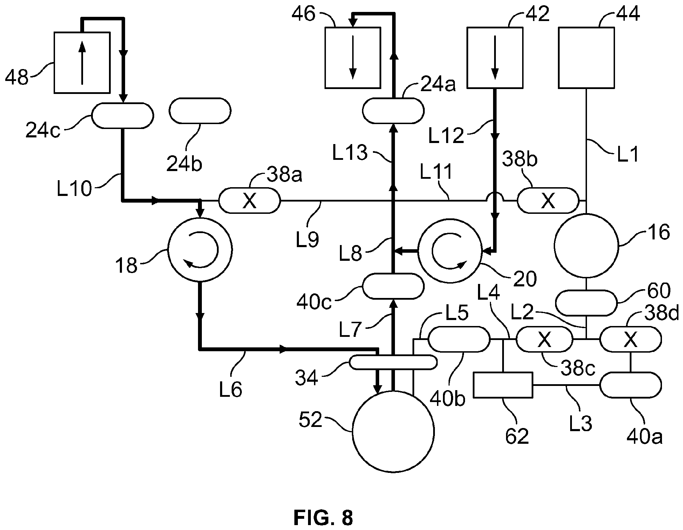

Once a total of one unit of whole blood has been drawn into the fluid flow circuit 12, the controller may transition the procedure to an "air flush" stage, which is shown in Fig. 8. During the air flush stage, air from the plasma collection container 48 (which was conveyed there during the blood prime stage) is used to recover the contents of the processing chamber 52 (which may be primarily red blood cells) to reduce product loss.

In the illustrated embodiment, the whole blood pump 16 is deactivated, while the plasma pump 18 is operated in a reverse direction (with respect to its direction of operation up to this stage of the procedure). This draws the air from the plasma collection container 48 and into line L10. Valve 38a remains closed, while clamp 24c remains open, which directs the air through line L10, into and through line L6, and into the processing chamber 52 via the plasma outlet port. On account of the air flowing through the plasma outlet port, it will enter the processing chamber 52 at the low-G side. As additional air is introduced into the processing chamber 52, it will move from the low-G wall towards the high-G wall, thus displacing any liquid content through the red blood cell outlet port at the high-G side and into line L7. During this stage, the centrifuge 22 may be operated at a slower rate (e.g., in the range of approximately 1,000-2,000 rpm) to decrease the risk of an air blockage (as during the blood prime stage).

The additive pump 20 continues its operation, drawing additive solution from the additive solution container 42 and through line L12, to be mixed with the contents of the processing chamber 52 flowing through line L7 at the junction of the two lines L7 and L12. The mixture continues flowing into and through line L8, through line L13 and open clamp 24a, and into the red blood cell collection container 46. The air flush stage continues until all of the air is removed from the plasma collection container 48. In one exemplary embodiment, the weight of the plasma collection container 48 may be monitored by a weight scale, with an empty plasma collection container 48 being detected when the weight is at or below a threshold value. Other approaches may also be employed to determine when to end the air flush stage, such as using the optical sensor 34 to detect plasma flowing through line L6.

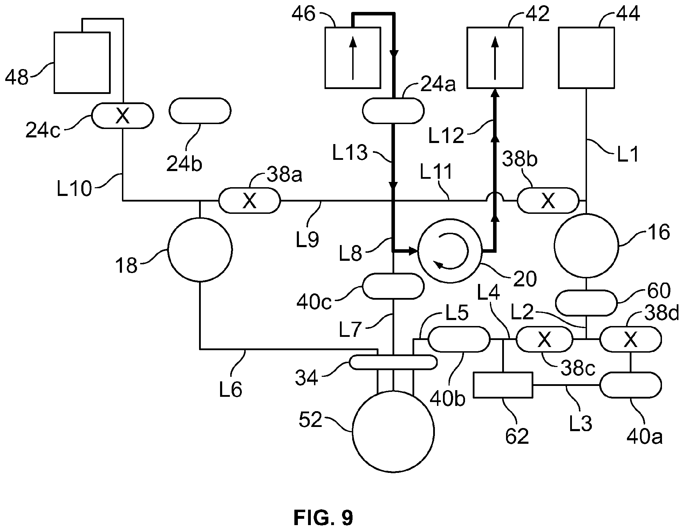

Once the air flush stage is complete, the procedure may transition to an air evacuation stage, as shown in Fig. 9. During the air evacuation stage, the red blood cell collection container 46 is "burped" to remove all residual air for storage (just as air was removed from the plasma collection container 48 during the air flush stage). This is done by reversing the direction of operation of the additive pump 20, closing clamp 24c, and stopping operation of the plasma pump 18. The additive pump 20 draws air out of the red blood cell collection container 46, through line L13 and open clamp 24a, into and through line L8, and then into and through line L12, with the air ending up in the additive solution container 42. While Fig. 9 shows the air being evacuated from the red blood cell container 46 to the additive solution container 42, it is within the scope of the present disclosure for all or a portion of the air to be directed to a different location of the fluid flow circuit 12 (e.g., into the processing chamber 52 and/or into the whole blood container 44, if provided).

The air evacuation stage will continue until all of the air is removed from the red blood cell collection container 46, which may be determined (for example) by detecting a change in the weight of the red blood cell collection container 46 (e.g., using a weight scale).

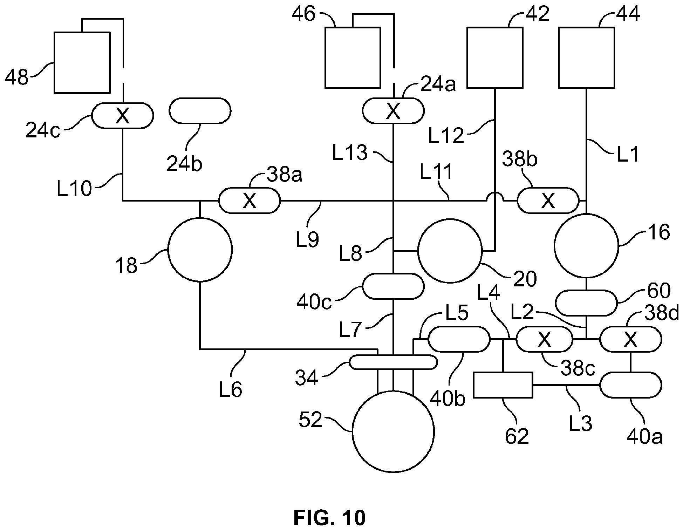

Upon completion of the air evacuation stage, any of a number of post-processing stages may be executed. For example, Fig. 10 shows a "sealing" stage in which all of the clamps and valves are closed and all of the pumps are deactivated. The line L13 connected to the red blood cell collection container 46 and the line L10 connected to the plasma collection container 48 are sealed and (optionally severed) for storage of the plasma and red blood cell products. If lines L10 and L13 are severed, the plasma collection container 48 and the red blood cell collection container 46 may be stored, while the remainder of the fluid flow circuit 12 is disposed of. Lines L10 and L13 may be sealed (and optionally severed) according to any suitable approach, which may include being sealed by RF sealers incorporated or associated with clamps 24a and 24c, for example. In another embodiment, the fluid flow circuit 12 may be removed from the processing device 10, with lines L10 and L13 being sealed (and optionally severed) using a dedicated sealing device.

It should be understood that the processing device 10 shown in Fig. 1, the fluid flow circuit 12 shown in Figs. 2 and 3, and the procedure shown in Figs. 4-10 are merely exemplary of a system and procedure that may employ the techniques and principles described herein. Indeed, differently configured blood separation systems and separation procedures including different steps may employ the techniques and principles described herein without departing from the scope of the present disclosure.

### Aspects

Aspect 1. A blood processing device, comprising: a pump system; a valve system; a continuous-flow centrifuge; and a controller programmed to control the operation of the pump system, the valve system, and the centrifuge to execute a blood separation procedure comprising: pumping whole blood through a leukoreduction filter and into the centrifuge, separating the blood in the centrifuge into red blood cells and plasma, with an interface between the red blood cells and plasma located at an interface position within the centrifuge, and pumping at least a portion of the red blood cells and at least a portion of the plasma out of the centrifuge.

Aspect 2. The blood processing device of Aspect 1, wherein said pumping whole blood through the leukoreduction filter includes removing white blood cells and platelets from the whole blood.

Aspect 3. The blood processing device of Aspect 1, wherein said pumping whole blood through the leukoreduction filter includes removing white blood cells from the whole blood while allowing platelets to pass through the leukoreduction filter.

Aspect 4. The blood processing device of any one of the preceding Aspects, further comprising a pressure sensor configured to measure a pressure of whole blood being pumped through the leukoreduction filter.

Aspect 5. The blood processing device of Aspect 4, wherein the controller is further programmed to receive signals from the pressure sensor that are indicative of the pressure of the whole blood being pumped through the leukoreduction filter, determine that flow through the leukoreduction filter has become blocked based at least in part on the signals from the pressure sensor, and upon determining that flow through the leukoreduction filter has become blocked, control the valve system to cause the whole blood to bypass the leukoreduction filter.

Aspect 6. The blood processing device of Aspect 5, wherein the controller is further programmed to control the pump system to change the interface position when the whole blood is bypassing the leukoreduction filter.

Aspect 7. The blood processing device of Aspect 6, wherein the controller is further programmed to control the pump system to move the interface position to a more radially outward position within the centrifuge when the whole blood is bypassing the leukoreduction filter.

Aspect 8. The blood processing device of any one of the preceding Aspects, wherein the controller is further programmed to execute a blood prime stage of the blood separation procedure in which whole blood is conveyed through the leukoreduction filter and through the centrifuge without being separated into red blood cells and plasma within the centrifuge.

Aspect 9. The blood processing device of Aspect 8, wherein the controller is further programmed to control the valve system to cause a portion of the whole blood to bypass the leukoreduction filter during the blood prime stage.

Aspect 10. The blood processing device of any one of the preceding Aspects, wherein the controller is further programmed to execute an establish separation stage of the blood separation procedure in which whole blood is conveyed through the leukoreduction filter, the whole blood is separated into red blood cells and plasma within the centrifuge, and the red blood cells and plasma are pumped out of the centrifuge and recombined.

Aspect 11. A blood separation method, comprising: pumping whole blood through a leukoreduction filter and into a continuous-flow centrifuge; separating the blood in the centrifuge into red blood cells and plasma, with an interface between the red blood cells and plasma located at an interface position within the centrifuge; and pumping at least a portion of the red blood cells and at least a portion of the plasma out of the centrifuge.

Aspect 12. The blood separation method of Aspect 11, wherein said pumping whole blood through the leukoreduction filter includes removing white blood cells and platelets from the whole blood.

Aspect 13. The blood separation method of Aspect 11, wherein said pumping whole blood through the leukoreduction filter includes removing white blood cells from the whole blood while allowing platelets to pass through the leukoreduction filter.

Aspect 14. The blood separation method of any one of Aspects 11-13, further comprising measuring a pressure of whole blood being pumped through the leukoreduction filter.

Aspect 15. The blood separation method of Aspect 14, further comprising determining that flow through the leukoreduction filter has become blocked based on the pressure of the whole blood being pumped through the leukoreduction filter, and upon determining that flow through the leukoreduction filter has become blocked, causing the whole blood to bypass the leukoreduction filter.

Aspect 16. The blood separation method of Aspect 15, further comprising changing the interface position when the whole blood is bypassing the leukoreduction filter.

Aspect 17. The blood separation method of Aspect 16, wherein the interface position is moved to a more radially outward position within the centrifuge when the whole blood is bypassing the leukoreduction filter.

Aspect 18. The blood separation method of any one of Aspects 11-17, further comprising a blood prime stage in which whole blood is conveyed through the leukoreduction filter and through the centrifuge without being separated into red blood cells and plasma within the centrifuge.

Aspect 19. The blood separation method of Aspect 18, further comprising causing a portion of the whole blood to bypass the leukoreduction filter during the blood prime stage.

Aspect 20. The blood separation method of any one of Aspects 11-19, further comprising an establish separation stage in which whole blood is conveyed through the leukoreduction filter, the whole blood is separated into red blood cells and plasma within the centrifuge, and the red blood cells and plasma are pumped out of the centrifuge and recombined.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A blood processing device (10), comprising:
a pump system (16, 18, 20);
a valve system (24a-c, 38a-d);
a continuous-flow centrifuge (22); and
a controller programmed to control the operation of the pump system (16, 18, 20), the valve system (24a-c, 38a-d), and the centrifuge (22) to execute a blood separation procedure comprising:
pumping whole blood through a leukoreduction filter (62) and into the centrifuge (22),
separating the blood in the centrifuge (22) into red blood cells and plasma, with an interface between the red blood cells and plasma located at an interface position within the centrifuge (22), and
pumping at least a portion of the red blood cells and at least a portion of the plasma out of the centrifuge (22).

2. The blood processing device (10) of claim 1, wherein said pumping whole blood through the leukoreduction filter (62) includes removing white blood cells and platelets from the whole blood.

3. The blood processing device (10) of claim 1, wherein said pumping whole blood through the leukoreduction filter (62) includes removing white blood cells from the whole blood while allowing platelets to pass through the leukoreduction filter (62).

4. The blood processing device (10) of any one of the preceding claims, further comprising a pressure sensor (40a) configured to measure a pressure of whole blood being pumped through the leukoreduction filter (62).

5. The blood processing device (10) of claim 4, wherein the controller is further programmed to
receive signals from the pressure sensor (40a) that are indicative of the pressure of the whole blood being pumped through the leukoreduction filter (62),
determine that flow through the leukoreduction filter (62) has become blocked based at least in part on the signals from the pressure sensor (40a), and
upon determining that flow through the leukoreduction filter (62) has become blocked, control the valve system (24a-c, 38a-d) to cause the whole blood to bypass the leukoreduction filter (62).

6. The blood processing device (10) of claim 5, wherein the controller is further programmed to control the pump system (16, 18, 20) to change the interface position when the whole blood is bypassing the leukoreduction filter (62).

7. The blood processing device (10) of claim 6, wherein the controller is further programmed to control the pump system (16, 18, 20) to move the interface position to a more radially outward position within the centrifuge (22) when the whole blood is bypassing the leukoreduction filter (62).

8. The blood processing device (10) of any one of the preceding claims, wherein the controller is further programmed to execute a blood prime stage of the blood separation procedure in which whole blood is conveyed through the leukoreduction filter (62) and through the centrifuge (22) without being separated into red blood cells and plasma within the centrifuge (22).

9. The blood processing device (10) of claim 8, wherein the controller is further programmed to control the valve system (24a-c, 38a-d) to cause a portion of the whole blood to bypass the leukoreduction filter (62) during the blood prime stage.

10. The blood processing device (10) of any one of the preceding claims, wherein the controller is further programmed to execute an establish separation stage of the blood separation procedure in which whole blood is conveyed through the leukoreduction filter (62), the whole blood is separated into red blood cells and plasma within the centrifuge (22), and the red blood cells and plasma are pumped out of the centrifuge (22) and recombined.

11. A blood separation method, comprising:
pumping whole blood through a leukoreduction filter (62) and into a continuous-flow centrifuge (22);
separating the blood in the centrifuge (22) into red blood cells and plasma, with an interface between the red blood cells and plasma located at an interface position within the centrifuge (22); and
pumping at least a portion of the red blood cells and at least a portion of the plasma out of the centrifuge (22).

12. The blood separation method of claim 11, further comprising
measuring a pressure of whole blood being pumped through the leukoreduction filter (62),
determining that flow through the leukoreduction filter (62) has become blocked based on the pressure of the whole blood being pumped through the leukoreduction filter (62), and
upon determining that flow through the leukoreduction filter (62) has become blocked, causing the whole blood to bypass the leukoreduction filter (62).

13. The blood separation method of claim 12, further comprising changing the interface position when the whole blood is bypassing the leukoreduction filter (62), preferably with the interface position being moved to a more radially outward position within the centrifuge (22) when the whole blood is bypassing the leukoreduction filter (62).

14. The blood separation method of any one of claims 11-13, further comprising a blood prime stage in which whole blood is conveyed through the leukoreduction filter (62) and through the centrifuge (22) without being separated into red blood cells and plasma within the centrifuge (22), preferably with a portion of the whole blood bypassing the leukoreduction filter (62) during the blood prime stage.

15. The blood separation method of any one of claims 11-14, further comprising an establish separation stage in which whole blood is conveyed through the leukoreduction filter (62), the whole blood is separated into red blood cells and plasma within the centrifuge (22), and the red blood cells and plasma are pumped out of the centrifuge (22) and recombined.
